(19)

Europäisches
Patentamt
European
Patent Office
Office européen
des brevets

(11) **EP 4 296 675 A1**

(12) **EUROPEAN PATENT APPLICATION**
published in accordance with Art. 153(4) EPC

(43) Date of publication:
**27.12.2023 Bulletin 2023/52**

(21) Application number: **21926870.3**

(22) Date of filing: **03.06.2021**

(51) International Patent Classification (IPC):
**G01N 33/68** (2006.01)   **C07K 16/24** (2006.01)
**C07K 14/005** (2006.01)

(52) Cooperative Patent Classification (CPC):
**C07K 14/005; C07K 16/24; G01N 33/68**

(86) International application number:
**PCT/KR2021/006975**

(87) International publication number:
**WO 2022/177071 (25.08.2022 Gazette 2022/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.02.2021 KR 20210020546**

(71) Applicant: **Boditech Med Inc.
Chuncheon-si, Gangwon-do 24398 (KR)**

(72) Inventors:
• **CHOI, Dong Hwan
Chuncheon-si, Gangwon-do 24448 (KR)**
• **KWON, Minsuk
Chuncheon-si, Gangwon-do 24413 (KR)**
• **BYUN, Hee Jung
Chuncheon-si, Gangwon-do 24209 (KR)**

(74) Representative: **Louis Pöhlau Lohrentz
Patentanwälte
Postfach 30 55
90014 Nürnberg (DE)**

(54) **INFORMATION PROVISION METHOD FOR EXAMINING ACTIVE IMMUNITY BY USING PRODUCTION OF NEUTRALIZING ANTIBODIES AND INTERFERON GAMMA**

(57) The present invention relates to an information provision method for examining active immunity by rapidly confirming, through a cell-mediated immunity test, the production of neutralizing antibodies and interferon gamma after vaccination. The present invention can determine whether neutralizing antibodies and interferon gamma are produced after vaccination, more accurately and rapidly through a cell-mediated immunity test. The present invention comprises (a) preparing a plurality of biological samples; (b) preparing a first reagent containing a vaccine antigen against SARS-CoV-2 or a protein antigen expressed by the vaccine, and a second reagent containing a protein derived from SARS-CoV-2 other than the antigen contained in the first reagent; (c) preparing a plurality of mixed samples by mixing the biological samples with the reagents; (d) preparing a plurality of detection reagents including a conjugate containing a signal generating means and an anti-interferon gamma antibody; (e) preparing a plurality of analyte samples by adding the detection reagent to the mixed sample; and (f) loading the analyte samples into a plurality of lateral flow cartridges and measuring signals from the cartridges using a signal detector; wherein at least one of the detection reagents further include a complex composed of a receptor-binding domain (RBD) protein derived from the spike S1 protein of SARS-CoV-2 and a signal generating means for generating a detectable signal by binding to the receptor-binding domain protein; and biotinylated human angiotensin-converting enzyme 2 (hACE2).

FIG. 4

**Description**

**TECHNICAL FIELD**

The present invention relates to a method for providing information for active immune diagnosis by determining whether neutralizing antibodies and interferon gamma are produced.

**BACKGROUND ART**

SARS-CoV-2 infection (coronavirus disease 2019, COVID-19) is a virus first discovered in 2019 in Wuhan City, Hubei Province, China, and belongs to the family of coronaviruses (Coronaviridae). It is known as a virus similar to SARS-CoV (severe acute respiratory syndrome coronavirus) and MERS-CoV (Middle East respiratory syndrome coronavirus). Although the source and transmission route of SARS-CoV-2 have not yet been clearly identified, it is likely to have been transmitted through contact with bats in Wuhan. Also, infection by close contact between people has been reported.

Recently, vaccine research for the prevention and treatment of SARS-CoV-2 infection is active, and various vaccines are expected to be distributed in the near future. Therefore, there is a need for a method to determine whether vaccine recipients need to be re-vaccinated by checking whether neutralizing antibodies are well formed, or to determine whether vaccination is necessary for a person with immunity formed through the production of neutralizing antibodies due to natural infection.

Interferon gamma (IFN-$\gamma$) is known to be very important for the recognition of microorganisms by innate immune cells and the development of cell-mediated immunity against intracellular pathogens in the initial host defense (Schroder et al., J Leukoc Biol. 2004 Feb;75(2):163-89). In particular, since T cells sensitive to a specific antigen secrete IFN-$\gamma$ when re-stimulated by the same antigen, the degree of IFN-$\gamma$ secretion has been applied to the diagnosis of bacterial or viral infections such as Mycobacterium tuberculosis and Leishmania braziliensis (Pai et al. Ann Intern Med. 2008;149(3):177-184, Turgay N et al, Am J Trop Med Hyg. 2010 Oct;83(4):822-4).

On the other hand, neutralizing antibodies refer to antibodies that defend cells by neutralizing the biological effects of pathogens or infectious particles when they penetrate the body. In order to determine whether active immunity is formed by a vaccine, it is necessary to check whether neutralizing antibodies are formed.

Accordingly, the present inventors determined that efficient inoculation for generating neutralizing antibodies is possible by determining the need for revaccination of the recipient by checking whether neutralizing antibodies and interferon gamma have been formed in the blood of the recipient after vaccination and the trend thereof.

**DETAILD DESCRIPTION OF THE INVENTION**

**TECHNICAL PROBLEM**

The present invention has been derived from the above needs. The present inventors have developed a method for providing information for active immune diagnosis, which can quickly check whether neutralizing antibodies and interferon gamma are produced after vaccination through a cell-mediated immunity test.

**TECHNICAL SOLUTION**

In one aspect, the present invention relates to a method of providing information for active immune diagnosis, the method comprising the steps of: (a) preparing a plurality of biological samples; (b) preparing a first reagent containing a vaccine antigen against SARS-CoV-2 or a protein antigen expressed by the vaccine, and a second reagent containing a protein derived from SARS-CoV-2 other than the antigen contained in the first reagent; (c) preparing a plurality of mixed samples by mixing the biological samples with the reagents; (d) preparing a plurality of detection reagents including a conjugate containing a signal generating means and an anti-interferon gamma antibody; (e) preparing a plurality of analyte samples by adding the detection reagent to the mixed sample; and (f) loading the analyte samples into a plurality of lateral flow cartridges and measuring signals from the cartridges using a signal detector; wherein at least one of the detection reagents further include a complex composed of a receptor-binding domain (RBD) protein derived from the spike S1 protein of SARS-CoV-2 and a signal generating means for generating a detectable signal by binding to the receptor-binding domain protein; and biotinylated human angiotensin-converting enzyme 2 (hACE2).

Preferably, the step (b) includes additionally preparing a third reagent containing Nil and a fourth reagent containing mitogen.

Preferably, the lateral flow cartridge in the step (f) comprises a strip including a sample inlet for injecting an analyte sample; a first test line with an anti-interferon gamma antibody immobilized; a second test line with streptavidin immobilized; and a control line with capture agent immobilized.

Preferably, the biological sample is whole blood, blood cells, or lymph fluid.

Preferably, the signal generating means may be an enzyme used for color development such as horseradish peroxidase (HRP) or a fluorescent material such as Europium (III) chelate nanoparticle (EuNP).

## TECHNICAL EFFECTS

The present invention can provide a lateral flow test method for determining whether interferon gamma and neutralizing antibodies are formed.

The present invention can provide information for active immunoassay by measuring whether interferon gamma and neutralizing antibodies are formed.

The present invention can also measure whether interferon gamma is formed in the blood of a recipient after vaccination, and directly determine whether stable neutralizing antibodies (i.e., active immunity) have been produced through the cellular immune process, thereby quickly determining whether the recipient needs to be revaccinated. In addition, the present invention improves the efficiency of immunization and prevents unnecessary waste of social funds due to unnecessary revaccination.

## BRIEF DESCRIPTION OF THE DRAWINGS

FIG. 1 illustrates a first reagent to a fourth reagent according to an embodiment of the present invention.

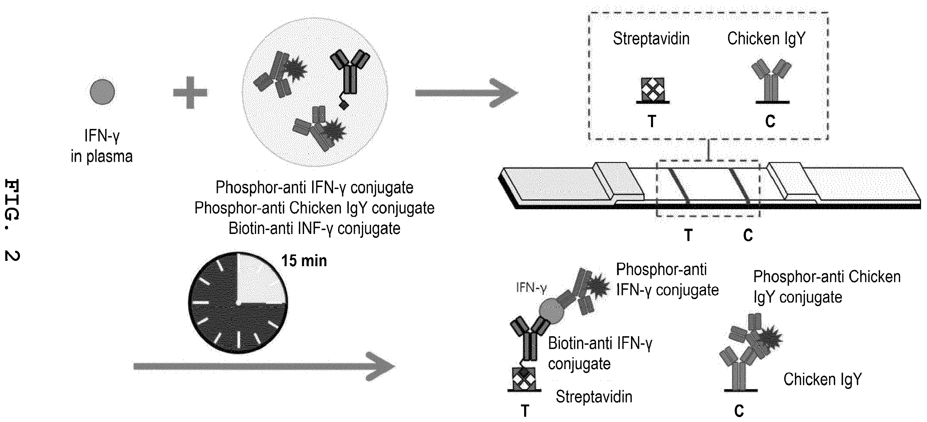

FIG. 2 illustrates a method for detecting interferon gamma according to an embodiment of the present invention.
FIG. 3 illustrates a method of detecting neutralizing antibodies according to an embodiment of the present invention.

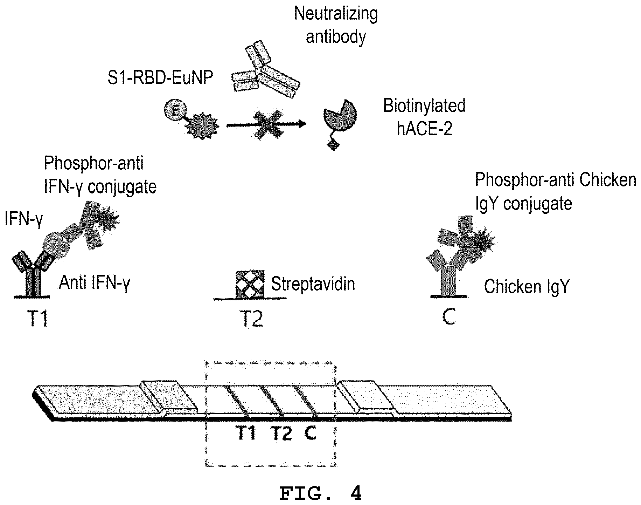

FIG. 4 illustrates a lateral flow test method for determining whether interferon gamma and neutralizing antibodies are formed according to an embodiment of the present invention.

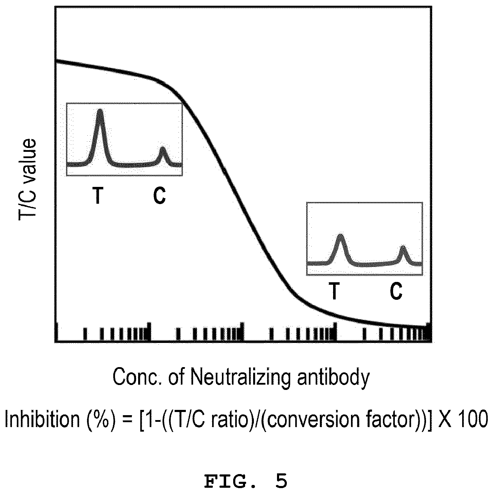

FIG. 5 illustrates a calibration curve for determining whether neutralizing antibodies are formed, according to an embodiment of the present invention.

## BEST MODE FOR ACCOMPLISHING THE INVENTION

The present invention uses a lateral flow test method to determine whether interferon gamma and neutralizing antibodies are formed in a biological sample.

An embodiment of the present invention, comprises the steps of: (a) preparing a plurality of biological samples; (b) preparing a first reagent containing a vaccine antigen against SARS-CoV-2 or a protein antigen expressed by the vaccine, and a second reagent containing a protein derived from SARS-CoV-2 other than the antigen contained in the first reagent; (c) preparing a plurality of mixed samples by mixing the biological samples with the reagents; (d) preparing a plurality of detection reagents including a conjugate containing a signal generating means and an anti-interferon gamma antibody; (e) preparing a plurality of analyte samples by adding the detection reagent to the mixed sample; and (f) loading the analyte samples into a plurality of lateral flow cartridges and measuring signals from the cartridges using a signal detector; wherein at least one of the detection reagents further include a complex composed of a receptor-binding domain (RBD) protein derived from the spike S1 protein of SARS-CoV-2 and a signal generating means for generating a detectable signal by binding to the receptor-binding domain protein; and biotinylated human angiotensin-converting enzyme 2 (hACE2).

The detection buffer may further include a detector labeled by a signal generating means, wherein the detector may be an anti-chicken IgY antibody, an anti-Rabbit IgG antibody, a BSA, an anti-NusA, or the like.

The neutralizing antibody may be a neutralizing antibody specific to the SARS-CoV-2 virus.

As used herein, the term "biological sample" refers to material taken from a person who has been vaccinated against SARS-CoV-2 and may be a liquid material or liquid-like fluid material, such as blood, blood cells, lymphatic fluid, saliva, urine, sweat, interstitial or intracellular fluid, or material extracted therefrom. Blood includes, but is not limited to, whole blood, plasma or serum, or blood, plasma or serum that has undergone a prescribed treatment (e.g., anti-clotting). The biological sample may be used with or without manipulation.

As used herein, the term "signal generating means" refers to a substance that generates a signal by binding to an antigen, antibody, or protein, and includes, but is not limited to, enzymes or proteins such as horseradish peroxidase (HRP), europium nanoparticle (EuNP), alkaline phosphatase (AP), glucose oxidase (GO), glutathione S-transferase (GST), maltose binding protein (MBP), acetylcholinesterase, alkaline phosphatase, $\beta$-D-galactosidase, $\beta$-lactamase, and lysozyme; fluorescent dyes such as fluorescein, isothiocyanate, rhodamine, phycoerythrin, phycocyanin, allophycocyanin, o-phthaldehyde, fluorescein, $Eu^{3+}$, $Eu^{3+}$ chelate or cryptate, and quantum dot; ligands such as biotin derivatives; luminescent materials such as acridinium esters, and isorhamnetin derivatives; microparticles such as colloidal gold, and colored latex; and radioactive isotopes such as $^{57}$Co, $^{3}$H, $^{125}$I, and $^{125}$I-Bonton Hunter reagents.

As used herein, the term "anti-interferon gamma antibody" refers to a monoclonal antibody specifically induced against interferon gamma.

As used herein, the term "spike S1 protein" refers to a protein that binds to a receptor protein on the surface of an infected cell and constitutes the spike protein, one of the structural proteins of the coronavirus.

As used herein, the term "receptor binding site" refers to a part located in the spike protein of the coronavirus, necessary to interact with the endogenous receptor to promote membrane fusion and delivery to the cytoplasm, and is an antibody neutralization site.

As used herein, the term "capture agent" refers to a binding agent (antibody or antigen) that specifically binds to an antigen, antibody, or protein present in an analyte sample, such as chicken IgY, Rabbit IgG, anti-BSA, and NusA etc.

The lateral flow cartridge may include, but is not limited to, a strip comprising a sample inlet for injecting an analyte sample; a first test line with an anti-interferon gamma antibody immobilized; a second test line with streptavidin immobilized; and a control line with capture agent immobilized.

Hereinafter, embodiments of the present invention will be described in detail with reference to the accompanying drawings and related descriptions. However, the following embodiments are only illustrative of this invention, and this invention is not limited by the following embodiments.

Exemplary embodiments

1. Preparing a biological sample

Blood is taken from a person to determine whether neutralizing antibodies to SARS-CoV-2 or interferon gamma are formed. The person may be a person vaccinated against SARS-CoV-2, a person infected with SARS-CoV-2, or a person who is not infected with SARS-CoV-2 and has not been vaccinated. The amount of blood collected is 10 to 50 ul.

2. Preparing reagents

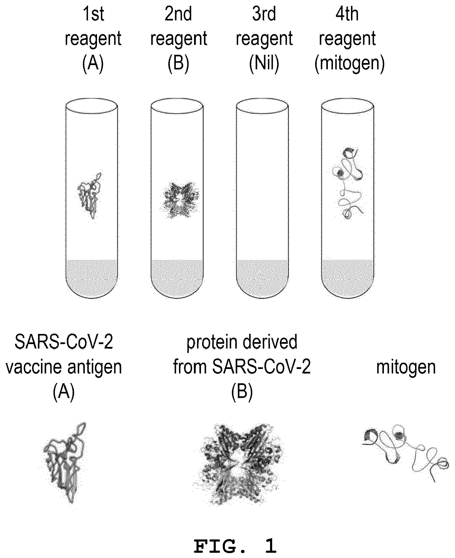

A first reagent containing a vaccine antigen against SARS-CoV-2 or a protein antigen expressed by the vaccine is prepared. In addition, a second reagent containing a protein derived from SARS-CoV-2 other than the antigen contained in the first reagent, for example, S protein, E protein, M protein, N protein, or Nucleocapsid protein, is prepared. In addition, a third reagent containing Nil and a fourth reagent containing mitogen are prepared. FIG. 1 shows tubes containing the four reagents.

3. Preparing mixed samples

The biological sample is mixed with the first to fourth reagents using a dispensing device to prepare a plurality of mixed samples. Here, the dispensing device may use the device disclosed in Korean Patent Registration No. 10-2178336.

When the biological sample from a vaccinated person or a person infected with SARS-CoV-2 is mixed with the first reagent, interferon gamma is formed. When the biological sample of a person infected with SARS-CoV-2 is mixed with the second reagent, interferon gamma is formed.

4. Preparing detection reagents

A plurality of detection reagents including anti-interferon gamma antibody conjugates labeled by EuNP are prepared. At least one of detection reagents include a complex composed of a receptor-binding domain (RBD) protein derived from the spike S1 protein of SARS-CoV-2, and a signal generating means such as EuNP; biotinylated human angiotensin-converting enzyme 2 (hACE2); and a conjugate of anti-chicken IgY and EuNP phosphor.

5. Preparing analyte samples

The detection reagent is mixed with the mixed sample to form a plurality of analyte samples.

Here, interferon gamma present in the mixed sample combines with the anti-interferon gamma antibody labeled by EuNP to form an interferon gamma complex.

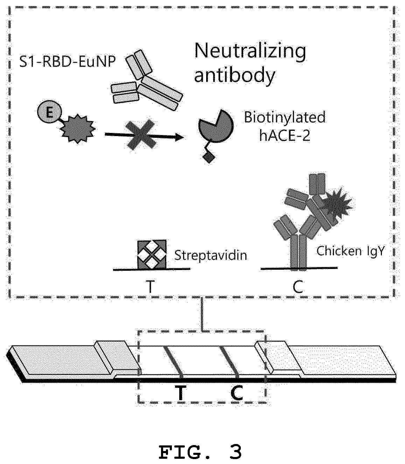

Further, as shown in FIG. 3, the neutralizing antibodies present in the mixed sample interfere with the binding of a receptor-binding domain (RBD) complex derived from a spike protein of SARS-CoV-2 labeled by a signal generating means, such as EuNP, to biotinylated human angiotensin-converting enzyme 2 (hACE2), thereby preventing the formation of a complex of RBD and hACE2.

6. Preparing lateral flow cartridges

A plurality of lateral flow cartridges comprising a strip are prepared. The strip comprises a sample inlet into which an analyte sample is injected; a first test line with an anti-interferon gamma antibody immobilized, at a location spaced apart from the sample inlet; a second test line with streptavidin immobilized, at a location spaced apart from the first test line; and a control line with chicken IgY immobilized.

7. Loading the analyte sample into the lateral flow cartridges

The analyte sample is loaded into the sample inlet. The analyte sample flows through the first test line, the second test line, and the control line along the strip. The interferon gamma complex present in the analyte sample is captured by the anti-interferon gamma antibody of the first test line. The Neutralizing antibodies in the analyte sample bind to the RBD protein in the analyte sample, and thus the binding between the RBD and the biotinylated human angiotensin-converting enzyme 2 (hACE2) is hindered, reducing the binding rate to streptavidin immobilized to the second test line. The anti-chicken IgY is captured by chicken IgY in the control line. This step takes 10 to 20 minutes, preferably 10 to 15 minutes.

8. Detecting a signal with a signal detector

The strength of the signal at each of the test line and the control line is measured through signal analysis using an optical system. More specifically, at the control line, a signal emitted from the conjugate of anti-chicken IgY and EuNP phosphor is detected to determine whether the lateral flow cartridge is normally operating. At the first test line, a signal emitted from the captured interferon gamma complex is detected. At the second test line, a signal emitted from a complex composed of the captured RBD proteins and HRP is detected. Since the neutralizing antibodies present in the analyte sample inhibit the capture of the RBD protein, the more neutralizing antibodies, the lower the intensity of the detected signal. A ratio of the signal strength (T value) measured on the second test line and the signal strength (C value) measured at the control line is calculated. The inhibition value is calculated using the calculated ratio and conversion factor (eigenvalue obtained through measurement of negative standard material) as shown in the equation below.

$$\text{Inhibition (\%)} = [1-((T/C \text{ ratio})/(\text{conversion factor}))] \times 100$$

At this time, it may be determined whether neutralizing antibodies are formed based on the cutoff value determined by the experiment. For example, if 20% is set as a cutoff value, neutralizing antibodies are determined to be formed if the inhibition value is greater than or equal to the cutoff value, and neutralizing antibodies are not determined to be formed if the inhibition value is less than the cutoff value. The relative inhibition value (Cut-off index: COI) based on this cut-off can be converted to an established international unit (IU) of antibody titer if the titer of the antibody neutralized by the vaccine is established and internationally accepted. The corresponding unit includes mIU/ml or IU/ml.

9. Meaning of signals from reagents

Meaning of the signal from the first reagent:

The first reagent contains a vaccine antigen, or a protein antigen expressed by the vaccine. Therefore, when the blood of a vaccinated person reacts with the first reagent, the secretion of interferon gamma increases by T cells present in the blood. In addition, when the blood of a person infected with SARS-CoV-2 reacts with the first reagent, the secretion of interferon gamma increases. If the level of interferon gamma is similar to that detected in the third reagent, it can be estimated to be the blood of a person who has not been vaccinated or infected with SARS-CoV-2.

Meaning of the signal from the second reagent:

The second reagent includes proteins derived from SARS-CoV-2 other than the antigens included in the first reagent. In the blood of the vaccinated person, there are no T cells that remember proteins derived from SARS-CoV-2 other than the antigens used in the vaccine. Therefore, interferon gamma is not detected even when the blood of the vaccinated person is mixed with the second reagent. However, in the blood of a person infected with SARS-CoV-2, there are T cells that remember proteins derived from SARS-CoV-2 other than antigens used in vaccines by infection. Therefore, interferon gamma is detected when the blood of the person infected with SARS-CoV-2 is mixed with the second reagent.

Meaning of the signal from the third reagent:

The third reagent contains Nil and serves as a negative control in the test. The signal generated from the third reagent increases the accuracy of the test result. For example, if the signal generated from the third reagent is greater than or equal to the cutoff value determined by the experiment, the test result is not accurate.

Meaning of the signal from the fourth reagnt:

The fourth reagent contains mitogen and serves as a positive control in the test. The signal generated in the fourth reagent increases the accuracy of the test result. For example, if the difference between the signal generated from the fourth reagent and the signal generated from the third reagent is less than or equal to the cutoff value determined by the experiment, the test result is not accurate.

10. Analysis of results

As shown in Table 1 below, in the case of a vaccinated person, the signal generated from the first reagent is strong, and the signal generated from the second reagent is weak. In the case of a person infected with the SARS-CoV-2 virus, both signals generated from the first reagent and the second reagent are strong. In the case of a person who has not been vaccinated and infected with the virus (a normal person), both signals generated from the first reagent and the second reagent are weak.

[Table 1

| Sample type | Interferon gamma secretion amount bv reagent type | | | |
| --- | --- | --- | --- | --- |
| | 1st reagent (vaccine antigen) | 2nd reagent (proteins derived from SARS-CoV-2 other than vaccine antigens) | 3rd reagent (Nil) | 4th reagent (mitogen) |
| Vaccine recipient | High | Low | Low | High |
| Person infected with SARS-CoV-2 | High | High | Low | High |
| Normal person | Low | Low | Low | High |

In addition, it is possible to determine whether neutralizing antibodies are formed by signals generated from the second test line and the control line.

The above results are finally provided as information for active immune diagnosis to a person with expertise, such as a specialist who conducts active immune diagnosis. A person with expertise, such as a specialist, may perform an active immune diagnosis based on the above results, and an example thereof is shown in Table 2.

[Table 2]

| Sample type | Confirmation of active immunity using a neutralizing antibody assay kit | |
| --- | --- | --- |
| Vaccine recipient | Positive (case 1) | Negative (case 2) |
| Person infected with SARS-CoV-2 | Positive (case 3) | Negative (case 4) |
| Normal person | - | Negative (case 5) |

For example, based on the result that the signal generated from the first reagent is strong, the signal generated from the second reagent is weak, and neutralizing antibodies are formed, a specialist may judge that active immunity by the vaccine is well formed and maintained in the vaccine recipient (case 1), or that the active immunity formed according to the efficacy of the vaccine or the immune ability of the vaccine recipient has disappeared (case 2). For example, based on the result that both the signal from the first reagent and the signal from the second reagent are strong and no neutralizing antibodies are formed, a specialist may judge that active immunity due to SARS-CoV-2 infection is well formed and maintained in the person infected with SARS-CoV-2 (case 3), or a specialist may judge that the active immunity formed according to the immune capacity of the SARS-CoV-2 infected person has disappeared (case 4), and may recommend vaccination. For example, based on the result that the signal generated from the first reagent is weak, the signal generated from the second reagent is weak, and neutralizing antibodies are formed, a specialist may determine that a normal person without vaccination or SARS-CoV-2 infection has no active immunity due to vaccination and SARS-CoV-2 infection (case 5), and may recommend vaccination.

The above description of the embodiments of the present invention is for illustrative purposes only, and those skilled in the art will understand that it can be easily transformed into other specific forms without changing the spirit or essential features of the present invention. Therefore, it should be understood that the embodiments described above are exemplary and not limited in all respects.

**Claims**

1. A method of providing information for active immune diagnosis, the method comprising the steps of:

   (a) preparing a plurality of biological samples;
   (b) preparing a first reagent containing a vaccine antigen against SARS-CoV-2 or a protein antigen expressed by the vaccine, and a second reagent containing a protein derived from SARS-CoV-2 other than the antigen contained in the first reagent;
   (c) preparing a plurality of mixed samples by mixing the biological samples with the reagents;
   (d) preparing a plurality of detection reagents including a conjugate containing a signal generating means and an anti-interferon gamma antibody;
   (e) preparing a plurality of analyte samples by adding the detection reagent to the mixed sample; and
   (f) loading the analyte samples into a plurality of lateral flow cartridges and measuring signals from the cartridges using a signal detector;

   wherein at least one of the detection reagents further include a complex composed of a receptor-binding domain (RBD) protein derived from the spike S1 protein of SARS-CoV-2 and a signal generating means for generating a detectable signal by binding to the receptor-binding domain protein; and biotinylated human angiotensin-converting enzyme 2 (hACE2).

2. The method of claim 1, wherein the step (b) includes additionally preparing a third reagent containing Nil and a fourth reagent containing mitogen.

3. The method of claim 1 or 2, wherein the lateral flow cartridge in the step (f) comprises a strip including a sample inlet for injecting an analyte sample; a first test line with an anti-interferon gamma antibody immobilized; a second test line with streptavidin immobilized; and a control line with capture agent immobilized.

4. The method of claim 1 or 2, wherein the biological sample is whole blood, blood cells, or lymph fluid.

5. The method of claim 1 or 2, wherein the signal generating means is horseradish peroxidase (HRP) or EuNP.

FIG. 1

FIG. 2

FIG. 3

FIG. 4

Conc. of Neutralizing antibody

Inhibition (%) = [1-((T/C ratio)/(conversion factor))] X 100

FIG. 5

## INTERNATIONAL SEARCH REPORT

International application No.

**PCT/KR2021/006975**

| A. | CLASSIFICATION OF SUBJECT MATTER |
| --- | --- |

**G01N 33/68**(2006.01)i; **C07K 16/24**(2006.01)i; **C07K 14/005**(2006.01)i

According to International Patent Classification (IPC) or to both national classification and IPC

| B. | FIELDS SEARCHED |
| --- | --- |

Minimum documentation searched (classification system followed by classification symbols)

G01N 33/68(2006.01); G01N 33/535(2006.01); G01N 33/543(2006.01); G01N 33/569(2006.01); G01N 33/58(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: SARS-CoV-2, COVID-19, 중화 항체 (neutralizing antibody), 인터페론 감마 (IFN-γ), T 세포 (T cell), 스파이크 (spike), 수용체 결합 부위 (receptor-binding domain, RBD), 인간 안지오텐신 전환 효소 2 (human angiotensin-converting enzyme 2, hACE2)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | TEBAS, P. et al. Safety and immunogenicity of INO-4800 DNA vaccine against SARS-CoV-2: A preliminary report of an open-label, Phase 1 clinical trial. EClinicalMedicine. 2021, vol. 31, 100689, inner pp. 1-9 (published online:24 December 2020). <br> See abstract, and inner pages 2, 3 and 5. | 1-5 |
| A | CN 111781354 A (BEIJING BIOSYSTEMS BIOLOGICAL TECHNOLOGY CO., LTD.) 16 October 2020 (2020-10-16) <br> See claim 1. | 1-5 |
| A | LAGAMAYO, E. N. et al. Association of interferon-gamma release assay and SARS-CoV-2 reverse transcriptase polymerase chain reaction test results in adults tested in a tertiary medical center. Journal of Clinical Microbiology and Biochemical Technology. 29 January 2021, vol. 7, no. 1, pp. 001-005. <br> See page 003. | 1-5 |
| A | CN 111273016 A (ZHEJIANG NEOGENE BIOTECHNOLOGY CO., LTD. et al.) 12 June 2020 (2020-06-12) <br> See claim 1. | 1-5 |

☑ Further documents are listed in the continuation of Box C.   ☑ See patent family annex.

| | |
| --- | --- |
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
| --- | --- |
| **10 November 2021** | **11 November 2021** |

| Name and mailing address of the ISA/KR | Authorized officer |
| --- | --- |
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2021/006975** |

C.    DOCUMENTS CONSIDERED TO BE RELEVANT

| Category* | Citation of document, with indication, where appropriate, of the relevant passages | Relevant to claim No. |
| --- | --- | --- |
| A | CN 112098644 A (JIANGSU MEDOMICS MEDICAL TECHNOLOGY CO., LTD.) 18 December 2020 (2020-12-18) <br> See claim 1. | 1-5 |

Form PCT/ISA/210 (second sheet) (July 2019)

**INTERNATIONAL SEARCH REPORT**
Information on patent family members

International application No.

**PCT/KR2021/006975**

| Patent document cited in search report | | | Publication date (day/month/year) | Patent family member(s) | | | Publication date (day/month/year) |
|---|---|---|---|---|---|---|---|
| CN | 111781354 | A | 16 October 2020 | CN | 111781354 | B | 01 December 2020 |
| CN | 111273016 | A | 12 June 2020 | CN | 111273016 | B | 15 June 2021 |
| CN | 112098644 | A | 18 December 2020 | None | | | |

Form PCT/ISA/210 (patent family annex) (July 2019)

**REFERENCES CITED IN THE DESCRIPTION**

**Patent documents cited in the description**

- KR 102178336

**Non-patent literature cited in the description**

- **SCHRODER et al.** *J Leukoc Biol.,* February 2004, vol. 75 (2), 163-89
- **PAI et al.** *Ann Intern Med.,* 2008, vol. 149 (3), 177-184
- **TURGAY N et al.** *Am J Trop Med Hyg.,* October 2010, vol. 83 (4), 822-4